# EUROPÄISCHE PATENTSCHRIFT

(11) **EP 0 648 549 B1**
(45) Veröffentlichungstag und Bekanntmachung des Hinweises auf die Patenterteilung: **19.11.1997**
(21) Anmeldenummer: 94115725.7
(22) Anmeldetag: 06.10.1994
(51) Int. Cl.: B08B 5/02, A61L 2/06

(54) **Reaktionskammer zur Dekontamination von Bekleidungs- und Ausrüstungsgegenständen**
Reaction chamber for decontamination of garment and equipment
Chambre de réaction pour décontamination de vêtements et d'équipement

(30) Priorität: 15.10.1993 DE 4335233
(43) Veröffentlichungstag der Anmeldung: 19.04.1995
(73) Patentinhaber: DORNIER GmbH, D-88039 Friedrichshafen (DE)
(72) Erfinder: Hellmuth, Paul, Dipl.-Ing., D-56322 Niederfell (DE); Klein, Norbert, Dipl.-Chem., D-29328 Fassberg (DE); Theis, Michael, Dipl.-Ing., D-56281 Emmelshausen (DE)
(74) Vertreter: Landsmann, Ralf, Dipl.-Ing.

(56) Entgegenhaltungen:
- EP-A- 0 138 688
- WO-A-83/02243
- DE-A- 3 119 664
- DE-A- 3 421 719
- PATENT ABSTRACTS OF JAPAN vol. 15, no. 105 (M-1092) 13. März 1991 & JP-A-03 001 029 (HITACHI LTD) 7. Januar 1991
- PATENT ABSTRACTS OF JAPAN vol. 14, no. 146 (C-704) 20. März 1990 & JP-A-02 017 067 (SUEHIRO SHOKAI KK) 22. Januar 1990

## Beschreibung

Die Erfindung betrifft eine Reaktionskammer mit den Merkmalen des Oberbegriffs des Anspruchs 1.

Eine derartige gattungsgemäße Reaktionskammer zeigt die DE-PS 34 21 719. Anstelle von Körben zur Aufnahme von beliebigen Gegenständen werden speziell ausgebildete Kleiderbügel zur Aufnahme von Schutzbekleidungen benutzt. Diese Kleiderbügel sind innen hohl ausgebildet und weisen Durchtrittsbohrungen auf. Über ein Druckrohr wird das Gasgemisch von oben den Kleiderbügeln zugeführt. Dieses Gasgemisch strömt aus den Durchtrittsbohrungen aus und bewirkt eine Innenbelüftung der Kleidungsstücke. Je nach der Position der Kleiderbügel auf der Förderschiene wechselt die zuvor beschriebene Innenbelüftung mit einer Außenbelüftung ab. In beiden Fällen wird das verbrauchte Gasgemisch genau unterhalb der jeweiligen Kleiderbügel wieder abgesaugt.Nach einer Reinigung des abgesaugten Gasgemisches wird dieses wieder dem Heißgaserzeuger zugeführt, so daß ein Kreislaufprozeß stattfindet. In Längsrichtung der Reaktionskammer sind mehrere Reaktionsstrecken nebeneinander angeordnet, um einen Parallelbetrieb zu erzielen. Eine räumliche Abtrennung zwischen den einzelnen Reaktionsstrecken ist nicht vorgesehen.

Die abwechselnd erfolgende Innen- und Außenbelüftung der Schutzanzüge ist kompliziert und aufwendig. Da nahezu das gesamte Heißgasgemisch im Kreislauf geführt wird, ist die Aufbereitung und Reinigung desselben aufwendig.

Bezüglich der Dekontaminationsgüte ist es von Nachteil, daß die nebeneinanderliegenden Reaktionsstrecken nicht voneinander getrennt sind und daß keine klare Trennung zwischen unreinen und reinen Abschnitten der Dekontaminationsstrecke vorgenommen wird.

Aufgrund der Bügelkonstruktion, die der Aufnahme von Bekleidungsgegenständen in der Innenbelüftung dient, ist die Dekontamination speziell auf die Entgiftung von Schutzanzügen ausgerichtet.

Aus der DE-PS 36 25 847 ist ferner eine Reaktionskammer mit einem Querstromventilator bekannt, um das Heißgasgemisch in der Reaktionskammer im Kreislauf umzuwälzen. Bei diesen Bekannten findet jedoch eine chargenweise Dekontamination statt, bei der die Kleidungsstücke während der Dekontamination ihren Platz in der Reaktionskammer beibehalten, was völlig im Gegensatz zu einer kontinuierlichen Dekontamination steht.

Aus der EP-A 0 138 688 ist eine stationäre Druckkammer zur Sterilisation von Gegenständen bekannt. Die Sterilisation erfolgt mit einem Gemisch aus Heißluft und Wasserdampf bei hohem Druck. Die Druckkammer wird chargenweise betrieben, wobei während der Sterilisation gasdurchlässige Körbe mit den zu sterilisierenden Gegenständen an Ort und Stelle verbleiben.

Der Erfindung liegt die Aufgabe zugrunde, für eine gattungsgemäße Reaktionskammer ein
- einfaches, kostengünstiges und
- im Hinblick auf die vorzunehmende BC-Dekontamination effizientes Gaszuführungs- und Gasabführungssystem zu schaffen, bei dem
- man variabel bezüglich der Art der zu dekontaminierten Gegenstände ist.

Diese Aufgabe wird durch die kennzeichnenden Merkmale des Anspruchs 1 gelöst.

Die mit der Erfindung erzielten Vorteile bestehen darin, daß das vom Gasgenerator erzeugte Heißgasgemisch in einfacher Weise auf die entlang einer Führung bewegten Körbe einwirkt. Zum einen wird das Gasgemisch durch die Reaktionsstrecke geleitet, dann abgesaugt und anschließend ins Freie abgeführt. Wegen der Ableitung ins Freie ist keine teure Aufbereitung des Gasstromes wie beim Bekannten erforderlich. Zum anderen werden die Körbe einer starken Querströmung ausgesetzt, bei der das Heißgasgemisch quer zur Längsströmung umgewälzt wird.

Die Reaktionsstrecke ist tunnelartig ausgebildet und im Querschnitt rundum geschlossen. Dies ergibt eine klare Trennung zwischen unreinen und reinen Bereichen. Hierdurch erreicht man in Verbindung mit der starken, auf die Körbe einwirkenden Querströmung eine hohe Dekontaminationsgüte und -effizienz.

In die gasdurchlässigen Körbe können Bekleidungsstücke und Ausrüstungsgegenstände gleichermaßen untergebracht werden. Die Querströmung sorgt unabhängig von der Art der zu dekontaminierenden Gegenstände für eine ausreichende Belüftung und damit für einen guten Warmeübergang auf die Gegenstände, was bei der BC-Dekontamination entscheidend ist.

Die Unteransprüche 2 - 6 stellen vorteilhafte Ausgestaltungen der Erfindung dar.

Die Erfindung wird nachfolgend anhand von in den Zeichnungen dargestellten Ausführungsbeispielen näher beschrieben. Hierbei zeigen
- Fig. 1: einen Sattelauflieger mit einer Einrichtung, die eine Reaktionskammer umfaßt, zur kontinuierlichen B- und/oder C-Dekontamination von Bekleidungs- und/oder Ausrüstungsgegenständen, in perspektivischer und teilweise aufgebrochener Darstellung;
- Fig. 2: eine Vorderansicht des in Fig. 1 dargestellten Sattelaufliegers, im Schnitt;
- Fig. 3: eine Draufsicht des in Fig. 1 dargestellten Sattelaufliegers, im Schnitt;
- Fig. 4: eine Seitenansicht des in Fig. 1 dargestellten Sattelaufliegers, im Schnitt;
- Fig. 5: eine schemahafte Draufsicht der in der Fig. 1 dargestellten Reaktionskammer, im Schnitt;
- Fign. 6 u. 7: jeweils eine alternative Ausbildung der Reaktionskammer, in schemahafter Draufsicht und im Schnitt.

### I. Übersicht über die Einrichtung zur Dekontamination

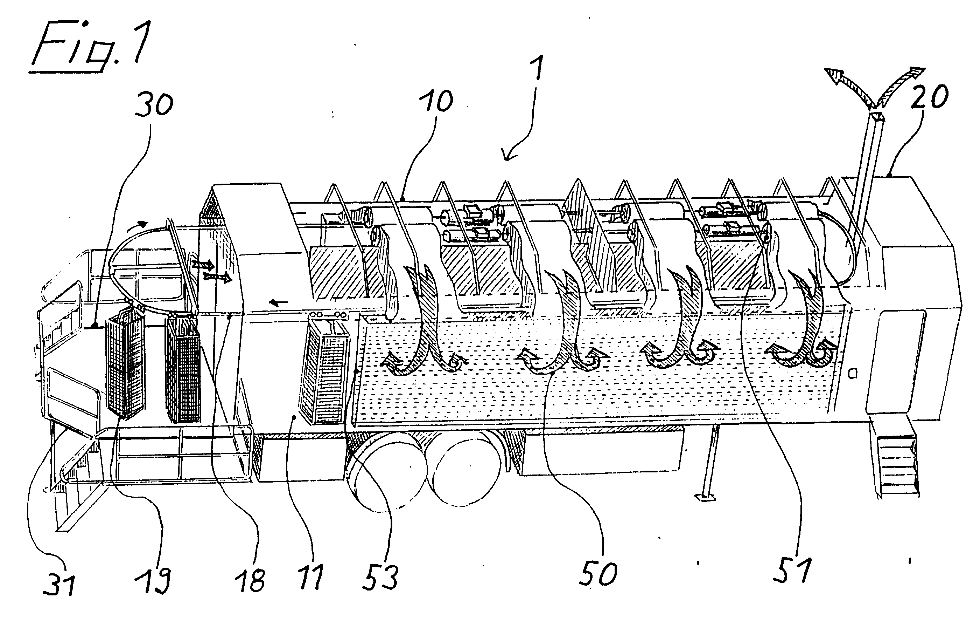

Die Fig. 1 zeigt einen Sattelauflieger 1, bei dem auf einem Fahrgestell eine Einrichtung zur kontinuierlichen Dekontamination von Bekleidungs- und/oder Ausrüstungsgegenständen aufgebaut ist. Diese Einrichtung ist dazu ausgelegt, um in einer Stunde 62 Bekleidungs- und Ausrüstungssätze zu dekontaminieren. Die Länge des Sattelaufliegers einschließlich der Sattelzugmaschine beträgt 16,5 m, die Breite 2,48 m und die Höhe 3,74 m, so daß öffentliche Straßen entsprechend handelsüblichen Sattelzügen befahren werden können.

Die Einrichtung zur Dekontamination ist in einen Geräteraum 20, eine Reaktionskammer 10 und eine Be- und Entladestation 30 gegliedert.

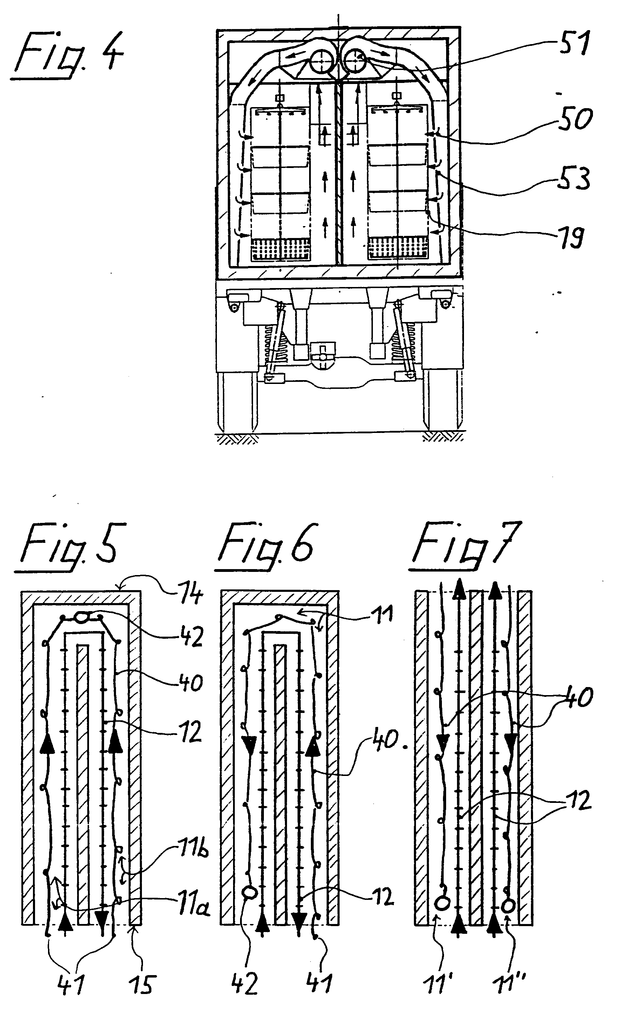

Der Geräteraum 20 (Fig. 3) umfaßt
- einen Vorratsbehälter 22 für Wasser (800 l) und einen Vorratsbehälter 21 für Kraftstoff (200 l), um einen autarken Betrieb über einen Zeitraum von ca. 3 Stunden zu ermöglichen;
- die gesamte elektrische Anlage mit dem von außen verlasteten Stromaggregat (6,5 kVA) 23;
- die Steuerungsanlage (SPS) 24, mit der man die Betriebsart vorgibt und die Anlage in Betrieb nimmt.

Die Reaktionskammer 10 (Fig. 3) enthält
- eine tunnelartige Reaktionsstrecke 11,
- ein Fördersystem für gasdurchlässige Körbe 19, welche die kontaminierten Gegenstände aufnehmen,
- zur BC-Dekontamination ein Gaszuführungs- und Gasahführungssystem für das Heißgasgemisch und
- zur A-Dekontamination Sprühköpfe zum Auftragen einer Dekontaminationslösung und Sprühköpfe zum Abwaschen dieser Lösung .

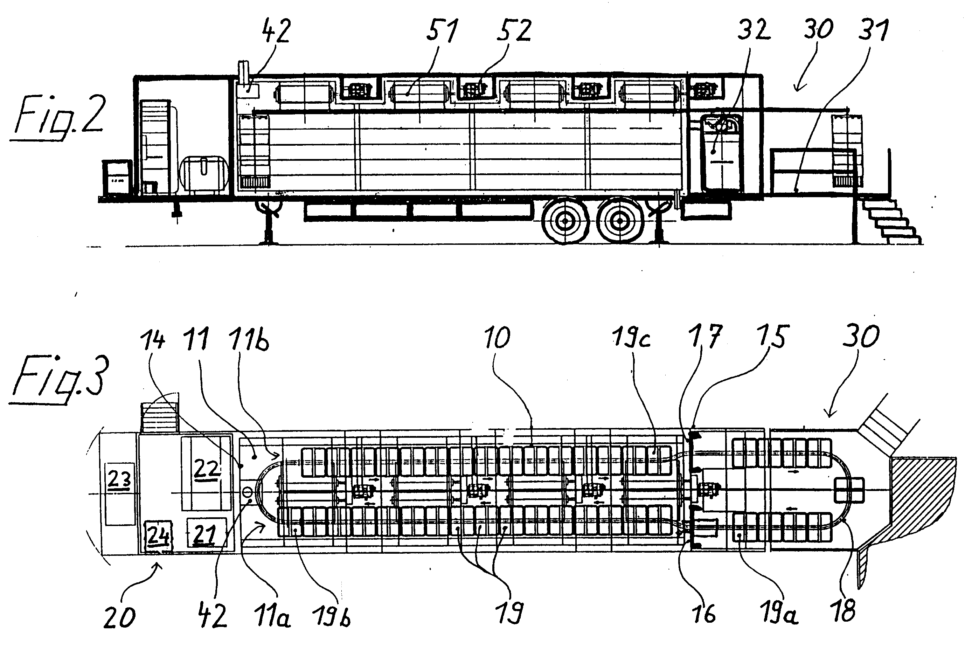

Die Be- und Entladestation 30 (Fig. 2) umfaßt
- eine Arbeitsbühne 31,
- einen Heißgaserzeuger 32 und
- Heizgeräte.

Wie in Fig. 1 dargestellt, kann die gesamte Einrichtung bei abgesetztem Sattelauflieger 1 in Betrieb genommen werden. Nach dem Aufbau, bei dem die Arbeitsbühne 31 herabgelassen wird und das Fördersystem für die Körbe 19 zusammengesetzt wird, ist die Betriebsart über die SPS-Steuerung zu wählen. Hierbei kann man zwischen einer BC- oder A-Dekontamination auswählen. Während die BC-Dekontamination mit einem Heißgasgemisch durchgeführt wird, erfolgt die A-Dekontamination mit Flüssigkeiten.

Für den Dekontaminationsbetrieb der kompletten Einrichtung werden zwei Personen benötigt, deren Hauptaufgaben sich auf das Beladen der Körbe 19 mit den kontaminierten Gegenständen und auf das Abladen des dekontaminierten Materials beziehen. Die Körbe 19 haben eine Höhe von 1,5 m, eine Breite von 0,5 m und eine Tiefe von 0,6 m, um die gesamte Bekleidung und Ausrüstung eines Soldaten oder einer im Zivilschutz arbeitenden Person aufzunehmen, was einem Gesamtvolumen von ca. 0,5 m³ und einem Gesamtgewicht von ca. 40 kg entspricht.

### II. BC-Dekontamination

Einzelheiten der Einrichtung werden zunächst bezüglich der BC-Dekontamination beschrieben.

Bevor der eigentliche Betrieb beginnt, wird mit dem Heißdampferzeuger 32 (Fig. 2) und mit zwei zusätzlichen Heizgeräten das gesamte System auf Betriebstemperatur gebracht. Die ideale Dekontaminationstemperatur liegt, wie Versuche zeigen, bei 170 °C, bei der einerseits chemische Kampf- oder Giftstoffe in unschädliche Substanzen zersetzt werden und bei der andererseits biologische Kampfstoffe zerstört werden. Bei temperaturempfindlichen Gegenständen kann die Temperatur auch bis hinab auf 130 °C abgesenkt werden. Das Dekontaminationsgemisch besteht zu 50 % aus den Abgasen eines Dieselölbrenners und zu 50 % aus Wasserdampf.

Die Reaktionsstrecke 11 (Fig. 3) ist tunnelartig ausgebildet und im Querschnitt rundum geschlossen. Ferner ist die Reaktionsstrecke 11 zweibahnig aufgebaut und setzt sich aus zwei nebeneinanderliegenden Teilabschnitten 11a und 11b zusammen, die in Längsrichtung der Reaktionskammer 10 verlaufen.

An der vorderen Stirnfläche 14 der Reaktionskammer 10 sind der erste und zweite Teilabschnitt 11a und 11b der Reaktionsstrecke 11 bogenförmig aneinandergereiht. An der hinteren Stirnfläche 15 dagegen, also an dem Anfang und Ende der Reaktionsstrecke 11, sind die Eingangs- und Ausgangstüren 16 und 17 angeordnet. Entlang der Reaktionsstrecke verläuft eine Führungsschiene 18 eines Fördersystems, entlang derer die gasdurchlässigen Körbe 19 zur Aufnahme der Gegenstände bewegbar sind. In dem Bereich der Be- und Entladestation 30 schließt sich die Führungsschiene 18 bogenförmig. Die einzelnen Körbe 19 bleiben immer im Eingriff mit der rundum geschlossenen Führungsschiene 18, (Fign. 1 und 3) und werden kreislaufförmig und kontinuierlich entlang einzelnen Stationen bewegt, die
- das Beladen auf der Be- und Entladestation 30,
- das Dekontaminieren in der Reaktionskammer 10 und
- das Entladen auf der Be- und Entladestation 30
umfassen.

Im einzelnen laufen hierbei folgende Arbeitsschritte ab (Fig. 3):
a) Zunächst wird der Korb 19b von dem ersten Teilabschnitt 11a entlang dem bogenförmigen, an der Stirnfläche 14 befindlichen Abschnitt zum zweiten Teilabschnitt 11b bewegt.
b) Anschließend wird die in dem ersten Teilabschnitt 11a befindliche Korbreihe um einen Platz vorgeschoben, um einen Leerplatz hinter der Eingangstür 16 zu schaffen.
c) Dann öffnet sich die Eingangstür 16, um den mit den zu dekontaminierenden Gegenständen gefüllten Korb 19a auf den zuvor im ersten Teilabschnitt 11a geschaffenen Leerplatz zu befördern.
d) Im Rahmen des Be- und Entladens werden die Körbe auf der Be- und Entladestation 30 um einen Platz vorgeschoben.
e) Dadurch kann der am Ende der Reaktionsstrecke 11 befindliche Korb 19c durch die Ausgangstür 17 auf die Be- und Entladestation 30 geführt werden.
f) Dann wird die im zweiten Teilabschnitt 11b befindliche Korbreihe um einen Platz vorgeschoben.

Anschließend beginnen die vorgenannten Schritte a) bis f) von vorne. Das Fördersystem für die Körbe ist an die SPS-Steuerung gekoppelt, so daß die zuvor genannten Arbeitsschritte automatisiert erfolgen.

Die innerhalb der Reaktionskammer 10 befindlichen Körbe 19 werden mit einem Heißgasgemisch beaufschlagt. Hierfür sorgt ein Gaszu- und Gasabführungssystem, welches innerhalb der Reaktionsstrecke 11 eine Gaslängsströmung und eine Gasquerströmung erzeugt.

Eingehend auf die Gaslängsströmung, (Fig. 3) münden Zuführungen, die an den Gasgenerator angeschlossen sind, an der hinteren Stirnfläche 15 der Reaktionskammer 10 in beide Teilabschnitte 11a und 11b ein. An der vorderen Stirnfläche 14 ist ein Absauggebläse 42 (Fign. 2 und 3) angeordnet. Dieses Absauggebläse 42 erzeugt einen Unterdruck und saugt dort das Heißgasgemisch an und bläst es ins Freie. Hierdurch wird in beiden Teilabschnitten 11a und 11b (Fig. 3) der Reaktionsstrecke 11 eine Gaslängsströmung erzeugt, die im ersten Teilabschnitt 11a gleichläufig und im zweiten Teilabschnitt 11b gegenläufig zur Förderrichtung der Körbe 19 verläuft. Weiterhin bewirkt der Unterdruck in der Reaktionskammer 11, daß beim Öffnen und Schließen der Türen 16 und 17 während des Be- und Entladens keine eventuell kontaminierten Gase austreten können.

Bezüglich der Gasquerströmung 50 (Fign. 1 und 4) sind auf dem Dach der Reaktionskammer mehrere Querstromgebläse 51 (Fign. 1, 2 und 4) angeordnet, die jeweils über ein Dieselaggregat 52 (Fig. 2) angetrieben werden. Jedes Querstromgebläse 51 (Fig. 4) bläst das Gasgemisch über ein Ausblaslochblech 53 (Fign. 1 und 4) aus, welches an einer der beiden Seitenwände der Reaktionsstrecke angeordnet ist. Das Lochblech 53 dient als Gasleitelement, um möglichst gleichmäßig die Körbe 19 zu belüften. Das Heißgasgemisch strömt seitlich quer zur Förderrichtung durch die Körbe und wird dann von dem Querstromgebläse 51 wieder angesaugt. Aufgrund der guten Belüftung der zu dekontaminierenden Gegenstände mit dem Heißgasgemisch erreicht man einen schnellen Wärmeübergang und damit eine schnelle Zerstörung der biologischen oder chemischen Kampfstoffe. Ferner bewirkt die im Kreislauf umlaufende Gasquerströmung eine hohe Ausnutzung des Heißgasgemisches.

Die schematisierten Figuren 5 - 7 verdeutlichen den Zusammenhang zwischen
- dem Verlauf der Reaktionsstrecke innerhalb der Reaktionskammer und
- der Gaslängsströmung.

Während die Fig. 5 dem bisherigen Ausführungsbeispiel entspricht, stellen die Fig. 6 und 7 jeweils ein alternatives Auführungsbeispiel dar. Nachfolgend werden die Varianten einander gegenübergestellt.

Die Fig. 5 stellt das bisher besprochene Ausführungsbeispiel dar, bei dem
a) Die Reaktionsstrecke aus zwei nebeneinanderliegenden, aneinandergereihten Teilabschnitten 11a und 11b besteht;
b) jeder Teilabschnitt 11a und 11b an der hinteren Stirnfläche 15 der Reaktionskanmer mit einer Zuführung 41 für das Heißgasgemisch versehen ist;
c) an der vorderen Stirnfläche 14 das Absauggebläse 42 angeordnet ist;
d) sich aufgrund b) und c) in jedem Teilabschnitt 11a und 11b die gezeigte Gaslängsströmung 40 ergibt, die im ersten Teilabschnitt 11a gleichläufig und im zweiten Teilabschnitt 11b gegenläufig zur Förderrichtung 12 der Körbe verläuft.

Als Unterschied zu dem bisherigen Ausführungsbeispiel zeigt die Variante nach der Fig. 6, daß
- das Heißgasgemisch nur am Ende der Reaktionsstrecke 11 über 41 zugeführt wird;
- das Absauggebläse 42 am Anfang der Reaktionsstrecke 11 angeordnet ist;
- die Gaslängsströmung 40 über nahezu (außer kurzes Stück an der Eingangstür) die gesamte Reaktionsstrecke 11 hinweg entgegen der Förderrichtung 12 der Körbe verläuft.

Der wesentliche Nachteil gegenüber dem ersten Ausführungsbeispiel besteht darin, daß-
- die Temperatur des Heißgasgemisches mit größer werdendem Weg der Gase entlang der Reaktionsstrecke 11 aufgrund der Wärmeverluste immer geringer wird, wodurch sich die Effizienz des Dekontaminierens verschlechtert.

Anders als beim ersten Ausführungsbeispiel zeigt die Fig. 7, daß
- zwei Reaktionsstrecken 11' und 11''vorgesehen sind, um einen Parallelbetrieb zu ermöglichen;
- die Gaslängsströmung 40 entlang der gesamten Reaktionsstrecke 11' bzw. 11''entgegen der Förderrichtung 12 der Körbe gerichtet ist.

Der Hauptnachteil dieser dritten Ausführung liegt darin, daß die Körbe über eine zusätzliche Bahn zum Anfang der Reaktionsstrecke 11' bzw. 11'' zurückgeführt werden müssen.

### III. A-Dekontamination

Die Reaktionskammer 10 (Fig. 1) ist auch für die A-Dekontamination ausgelegt. Hierzu sind in einem ersten Abschnitt der Reaktionsstrecke 11 auf einem Sprührahmen Düsen zum Aufsprühen einer Dekontaminationslösung angeordnet. In einem hierauf folgenden Abschnitt der Reaktionsstrecke 11 sind ebenfalls Sprührahmen mit Düsen zum Abwaschen der Dekontaminationslösung mit einer Abwaschlösung (z. B. Wasser) plaziert. In einem letzten Abschnitt der Reaktionsstrecke 11 befinden sich Gebläse zur Trocknung mit Heißluft. Die Einspeisung der Dekontaminations- und der Abwaschlösung erfolgt jeweils über einen Außenanschluß. Zur Erzielung einer hohen Dekontaminationsgüte sollte ein Satz mit Bekleidungs- und Ausrüstungsgegenständen auf zwei Körbe 19 verteilt werden. Der Betriebsablauf für die A-Dekontamination wird wie bei der BC-Dekontamination durch die SPS-Steuerungsanlage unterstützt und erfolgt daher automatisiert.

## Patentansprüche

1. Länglich ausgebildete, auf ein Fahrgestell aufbaubare Reaktionskammer (10) einer mobilen Einrichtung zur kontinuierlichen B-und/oder C-Dekontamination von Bekleidungs- und/oder Ausrüstungsgegenständen mit einem Gemisch aus mindestens Heißluft und Wasserdampf, bestehend aus
a) einer Reaktionsstrecke (11), die tunnelartig und im Querschnitt rundum geschlossen ist und die
- einbahnig oder
- mehrbahnig in Form von nebeneinanderliegenden, aneinandergereihten Teilabschnitten (11a, 11b)
in Längsrichtung der Reaktionskammer (10) angeordnet ist
b) einem Fördersystem mit einer entlang der Reaktionsstrecke verlaufenden Führungsschiene (18), entlang derer Behälter (19) zur Aufnahme der Gegenstände bewegbar sind;
c) einer Eingangstür (16) am Anfang der Reaktionsstrecke (11) für den Einlaß der mit kontaminierten Gegenständen versehenen Behälter (19);
d) einer Ausgangstür (17) am Ende der Reaktionsstrecke (11) für den Auslaß der mit den dekontaminierten Gegenständen versehenen Behälter (19);
e) einem auf die bewegbaren Behälter (19) einwirkenden Gaszuführungs- und Gasabführungssystem für das Heißgasgemisch, mit
e1) mindestens einer Zuführung (41), die an einen Heißgasgenerator (32) anschließbar ist, und mindestens einem Absauggebläse (42), um verbrauchtes Gas über eine Abführung ins Freie zu leiten,
**dadurch gekennzeichnet**,
- daß gasdurchlässige Körbe (19) als Behälter für die zu dekontaminierenden Gegenstände eingesetzt werden;
- daß sich zwischen der oder den Zuführungen (41) und dem oder den Absauggebläsen (42) die gesamte Dekontaminationsstrecke (11) oder deren Teilabschnitte (11a, 11b) befinden, um eine Gaslängsströmung (40) zu erzeugen, die zumindest im letzten Teilabschnitt (11b) der Reaktionsstrecke (11) gegenströmig zur Förderrichtung (12) der Körbe (19) ist;
- daß mehrere Querstromgebläse (51) mit Gasleitelementen entlang der Reaktionsstrecke (11), zur Erzeugung einer im Kreislauf geführten, quer zur Reaktionsstrecke verlaufenden Gasquerströmung (50) angeordnet sind.

2. Reaktionskammer nach Anspruch 1, **dadurch gekennzeichnet**, daß die Querstromgebläse (51) und die zugehörigen Antriebsaggregate (52) auf dem Dach der Reaktionskammer (10) angeordnet sind.

3. Reaktionskammer nach Anspruch 1 oder 2, **dadurch gekennzeichnet**, daß die Gasleitelemente Lochbleche (53) zur Lenkung des Heißgasgemisches umfassen.

4. Reaktionskammer nach Anspruch 3, **dadurch gekennzeichnet**, daß die Lochbleche (53) entlang einer Seite der Reaktionsstrecke (11) angeordnet sind und daß im Betrieb die Querstromgebläse (51) das Heißgasgemisch durch diese Lochbleche (53) blasen.

5. Reaktionskammer nach einem der Ansprüche 1 bis 4, **dadurch gekennzeichnet**, daß
a) die Reaktionsstrecke (11) zweibahnig ist und aus einem ersten und zweiten Teilabschnitt (11a, 11b) beseht und demzufolge
a1) der erste und der zweite Teilabschnitt (11a, 11b) an einer ersten Stirnfläche (14) der Reaktionskammer (10) aneinandergereiht sind;
a2) an der zweiten Stirnfläche (15) der Anfang und das Ende der Reaktionsstrecke (11) bzw. die Eingangs- und Ausgangstür (16, 17) angeordnet sind;
b) an der zweiten Stirnfläche (15) sowohl am Anfang als auch am Ende der Reaktionsstrecke (11) die Zuführungen (41) für das Heißgasgemisch einmünden und an der ersten Stirnfläche (14) ein Absauggebläse (42) für beide Teilabschnitte (11a, 11b) angeordnet sind, so daß der sich im ersten Teilabschnitt (11a) bildende Gaslängsstrom (40) gleichläufig und der sich im zweiten Teilabschnitt (11b) bildende Gaslängsstrom gegenläufig zur Förderrichtung (12) der Körbe (19) verläuft.

6. Reaktionskammer nach einem der Ansprüche 1 bis 5, **gekennzeichnet durch** folgende Merkmale zu A-Dekontamination:
a) in einem ersten Abschnitt der Reaktionsstrecke (11) sind Sprührahmen mit Düsen zum Aufsprühen einer Dekontaminationslösung angeordnet,
b) in einem folgenden Abschnitt der Reaktionsstrecke (11) sind Sprührahmen mit Düsen zum Abwaschen der Dekontaminationslösung mit einer Abwaschlösung angeordnet;
c) in einem letzten Abschnitt der Reaktionsstrecke (11) sind Gebläse zur Trocknung mit Heißluft angeordnet.

## Claims

1. Reaction chamber (10) of a mobile arrangement, which reaction chamber is of elongate construction and is arranged to be mounted on a chassis, for the continuous B- and/or C-decontamination of articles of clothing and/or equipment using a mixture of at least hot air and water vapour, comprising
a) a reaction path (11) which is tunnel-like and in cross-section is fully closed and which is arranged in the longitudinal direction of the reaction chamber (10) as
- a single track or
- a plurality of tracks in the form of subsections (11a, 11b) ranged side by side;
b) a conveyor system having a guide rail (18) running along the reaction path and along which containers (19) for receiving the articles are movable;
c) an entry door (16) at the start of the reaction path (11) for admission of the containers (19) charged with contaminated articles;
d) an exit door (17) at the end of the reaction path (11) for discharge of the containers (19) charged with the decontaminated articles;
e) a gas supply and gas discharge system for the hot gas mixture, which system acts on the movable containers (19), having
e1) at least one supply (41), which is arranged to be connected to a hot gas generator (32), and at least one extraction fan (42) for conveying used gas by way of an outlet into the open air.
**characterised in that**
- gas-permeable baskets (19) are used as containers for the articles to be decontaminated;
- the entire decontamination path (11) or the subsections (11a, 11b) thereof is/are located between the supply or supplies (41) and the extraction fan or fans (42) in order to generate a longitudinal flow of gas (40) which at least in the last subsection (11b) of the reaction path (11) flows in the opposite direction to the conveying direction (12) of the baskets (19);
- several cross-flow fans (51) having gas-deflecting elements are arranged along the reaction path (11) to generate a cross-flow of gas (50) guided in a circulatory movement and running transversely to the reaction path.

2. Reaction chamber according to claim 1, **characterised in that** the cross-flow fans (51) and the associated operating units (52) are arranged on the roof of the reaction chamber (10).

3. Reaction chamber according to claim 1 or 2, **characterised in that** the gas-deflecting elements comprise perforated plates (53) for guiding the hot gas mixture.

4. Reaction chamber according to claim 3, **characterised in that** the perforated plates (53) are arranged along a side of the reaction path (11) and that in operation the cross-flow fans (51) blow the hot gas mixture through these perforated plates (53).

5. Reaction chamber according to any one of claims 1 to 4, **characterised in that**
a) the reaction path (11) is two-track and consists of a first and a second subsection (11a, 11b) and therefore
a1) the first and the second subsection (11a, 11b) are ranged side by side at a first end face (14) of the reaction chamber (10);
a2) the beginning and the end of the reaction path (11), respectively the entry and exit doors (16, 17), are arranged at the second end face (15);
b) the supplies (41) for the hot gas mixture discharge at the second end face (15) both at the beginning and at the end of the reaction path (11) and an extraction fan (42) for both subsections (11a, 11b) is arranged at the first end face (14), so that the longitudinal gas flow (40) developing in the first subsection (11a) runs in the same direction as and the longitudinal gas flow developing in the second subsection (11b) flows in the opposite direction from the conveying direction (12) of the baskets (19).

6. Reaction chamber according to any one of claims 1 to 5, **characterised by** the following features for A-decontamination:
a) spray frames with nozzles for spraying on a decontamination solution are arranged in a first section of the reaction path (11).
b) spray frames with nozzles for washing out the decontamination solution with a rinsing solution are arranged in a following section of the reaction path (11);
c) fans for drying with hot air are arranged in a last section of the reaction path (11).

## Revendications

1. Chambre de réaction (10) allongée pouvant être montée sur un châssis roulant d'un dispositif mobile pour la décontamination B et/ou C en continu de vêtements et d'objets d'équipement avec un mélange au moins d'air chaud et de vapeur d'eau, comprenant
a) un parcours de réaction (11) en forme de tunnel à section transversale fermée tout autour et disposé,
- en une seule voie ou
- en plusieurs voies sous la forme de tronçons partiels (11a, 11b) juxtaposés;
dans la direction longitudinale de la chambre de réaction (10),
b) un système de transport avec un rail de guidage (18) qui s'étend le long du parcours de réaction et le long duquel peuvent être déplacés des récipients (19) pour la réception des objets;
c) une porte d'entrée (16) au début du parcours de réaction (11) pour l'entrée des récipients (19) chargés d'objets contaminés;
d) une porte de sortie (17) à la fin du parcours de réaction (11) pour la sortie des récipients (19) chargés des objets décontaminés;
e) un système d'amenée et d'évacuation de gaz pour le mélange de gaz chauds agissant sur les récipients (19) mobiles, avec
e1) au moins une arrivée (41) pouvant être raccordée à un générateur de gaz chaud (32), et au moins un ventilateur extracteur (42) pour évacuer les gaz usés vers l'extérieur par l'intermédiaire d'une sortie,
**caractérisée en ce**
- que des paniers (19) perméables aux gaz sont utilisés comme récipients pour les objets à décontaminer;
- qu'entre la ou les arrivée(s) (41) et le ou les ventilateur(s) extracteur(s) (42) se trouve l'ensemble du parcours de décontamination (11) ou les tronçons partiels (11a, 11b) de celui-ci, pour créer un écoulement de gaz longitudinal (40) qui est orienté, au moins dans le dernier tronçon partiel (11b) du parcours de réaction (11) à contre-courant par rapport à la direction de transport (12) des paniers (19);
- que plusieurs ventilateurs à courant transversal (51) avec des éléments directeurs de gaz sont disposés le long du parcours de réaction (11) pour produire un courant de gaz transversal (50) conduit en circuit fermé et orienté transversalement au parcours de réaction.

2. Chambre de réaction selon la revendication 1, caractérisée en ce que le ventilateur à courant transversal (51) et les groupes d'entraînement (52) associés sont disposés sur le toit de la chambre de réaction (10).

3. Chambre de réaction selon la revendication 1 ou 2, caractérisée en ce que les éléments directeurs de gaz comprennent des tôles perforées (53) pour le guidage du mélange de gaz chauds.

4. Chambre de réaction selon la revendication 3, caractérisée en ce que les tôles perforées (53) sont disposées le long d'un côté du parcours de réaction (11) et que, en service, les ventilateurs à courant transversal (51) soufflent le mélange de gaz chauds au travers desdites tôles perforées (53).

5. Chambre de réaction selon l'une des revendications 1 à 4, caractérisée en ce que
a) le parcours de réaction (11) est à deux voies et se compose d'un premier et d'un second tronçons partiels (11a, 11b) et que, en conséquence,
a1) les premier et second tronçons partiels (11a, 11b) sont disposés en ligne sur une première surface frontale (14) de la chambre de réaction (10);
a2) sur la seconde surface frontale (15) sont disposés le début et la fin du parcours de réaction (11) et respectivement les portes d'entrée et de sortie (16, 17);
b) dans la seconde surface frontale (15) au début aussi bien qu'à la fin du parcours de réaction (11) débouchent les arrivées (41) pour le mélange de gaz chauds et que sur la première surface frontale (14) est disposé un ventilateur extracteur (42) pour les deux tronçons partiels (11a, 11b), de sorte que le courant de gaz longitudinal (40) formé dans le premier tronçon partiel (11a) s'étend dans le même sens que la direction de transport (12) des paniers (19), et que le courant de gaz longitudinal formé dans le second tronçon partiel (11b) est orienté dans le sens opposé à cette direction de transport.

6. Chambre de réaction selon l'une des revendications 1 à 5, avec les caractéristiques suivantes pour la décontamination A :
a) dans une première partie du parcours de réaction (11) sont disposés des cadres de pulvérisation avec des buses pour la pulvérisation d'une solution de décontamination;
b) dans une partie suivante du parcours de réaction (11) sont disposés des cadres de pulvérisation avec des buses pour le lavage de la solution de décontamination au moyen d'un solution de lavage;
c) dans une dernière partie du parcours de réaction (11) sont disposés des ventilateurs pour le séchage à air chaud.
